# EUROPEAN PATENT APPLICATION

(11) **EP 2 075 341 A1**
(43) Date of publication of application: **01.07.2009**
(21) Application number: 07806736.0
(22) Date of filing: 05.09.2007
(51) Int. Cl.: C12Q 1/68, G01N 33/15, G01N 33/50, C12N 15/12

(54) **METHOD FOR STUDY, DETERMINATION OR EVALUATION BY GENE EXPRESSION ANALYSIS**

(30) Priority: 06.09.2006 JP 2006241400
(71) Applicant: Nippon Zoki Pharmaceutical Co., Ltd., Osaka-shi Osaka 541-0046 (JP)
(72) Inventor: NAIKI, Mitsuru, Kato-shi Hyogo 673-1461 (JP); OKADA, Tomoyuki, Kato-shi Hyogo 673-1461 (JP)
(74) Representative: HOFFMANN EITLE
(86) International application number: PCT/JP2007/067293
(87) International publication number: WO 2008/029838

(57) **Abstract**

There is provided a method for study, determination or evaluation of a disease condition due to SART stressing or a pharmacological activity, for example an analgesic activity, an autonomic imbalance-ameliorating activity or an anti-stress activity, of a test substance at the gene level. It is a method for study, determination or evaluation of a pharmacological activity of a test substance, especially an analgesic activity, an autonomic imbalance-ameliorating activity, or an anti-stress activity at the gene level by comprehensively analyzing the change in gene expression in a neural tissue after administration of a test substance in a SART-stressed animal. The method enables exploration of the substance that is effective against painful diseases, stress-related diseases, autonomic-imbalance or the like, determination and evaluation of efficacy of the substance, or analysis of a target gene of the substance

## Description

### TECHNICAL FIELD

The present invention relates to a method for study, determination or evaluation of a disease condition due to SART stressing or a pharmacological activity, for example an analgesic activity, an autonomic imbalance-ameliorating activity or an anti-stress activity, of a test substance at the gene level by analyzing the change in gene expression in a neural tissue after administration of a test substance in a SART stressed animal.

### BACKGROUND ART

By breeding an experimental animal for several days at a temperature for breeding environment changed every one hour during day and reduced at night, SART (specific alternation of rhythm in temperature) stress, in other words, a repeated cold stress, is loaded onto the animal, and SART stressed animals such as a mouse, rat and guinea pig and the like are prepared. A SART stressed animal is considered as a pathological model of pain hypersensitivity, autonomic imbalance, and stress condition that can be prepared in accordance with a method of Kita, et al. (Folia Pharmacologica Japonica, vol. 71, p 195, 1975). For example, in the case of rats, a temperature for breeding environment is changed at 24°C and -3°C every one hour from 10 a.m. to 5 p.m., then it is kept at -3°C from 5 p.m. to 10 a.m. of the next morning. Thus, the repetitive cold stress is loaded by breeding for 4 days or more where water and feed are freely taken by them thereupon the SART stressed rats are prepared. The temperature setting of as low as -3°C in the case of rats is changed to 4°C for mice and to 0°C for guinea pigs whereupon SART stressed mice and SART stressed guinea pigs are able to be prepared respectively.

In the SART stressed animals prepared as such, there have been known such characteristics that the repeated cold stress causes decrease in their pain threshold values, promotion of anxiety and depression, decrease in body weight, increase in release of CRH (corticotropin-releasing hormone), noradrenaline and IL-1β, and suppression of release of serotonin (5-HT).

In the meanwhile, an extract from inflamed tissues inoculated with vaccinia virus used in the present invention as a test substance contains a non-protein active substance that is isolated through extraction of a rabbit inflamed skin tissue inoculated with vaccinia virus. It has been known already that, in SART stressed animals, the extract from inflamed tissues inoculated with vaccinia virus has a variety of actions such as a suppressive action for lowering of pain threshold (pain sensitivity) (analgesic action), a suppressive action for promotion of release of CRH, noradrenaline and IL-1β and for suppression of release of serotonin (5-HT) and a suppressive action for body weight decrease (autonomic imbalance-ameliorating activity, an anti-stress activity) (see Non-Patent Documents 1 and 2). The titer determination of a pharmaceutical preparation that an extract from an inflamed rabbit skin inoculated with vaccinia virus is an effective ingredient (trade name: Neurotropin) is conducted by means of an analgesic effect test using the SART stressed animals, which is defined as a quantitative test therefor.

The preparation of an extract from inflamed rabbit skin inoculated with vaccinia virus is a very unique preparation which has been allowed to be used for a broad range of indications such as itch accompanies by skin diseases (such as eczema, dermatitis and urticaria), allergic rhinitis and sequelae of SMON such as coldness, paresthesia, and pain, in addition to painful diseases such as low back pain, neck-shoulder-arm syndrome, symptomatic neuralgia, periarthritis scapulohumeralis, degenerative arthritis deformans and post-herpetic neuralgia. Injection preparations for hypodermic, intramuscular and intravenous uses and tablet preparations have been approved to manufacture as ethical drugs and put into the market. In recent years, clinical tests thereof have been carried out in the United States for RSD (reflex sympathetic dystrophy, CRPS-type 1) which is an intractable neuropathic pain.

It has been also reported that the extract from inflamed tissues inoculated with vaccinia virus is effective for fibromyalgia (see Non-Patent Document 1). Although, causes and mechanisms of development of fibromyalgia are presumed to be, for the time being, psychological factors such as stress or the like, viral infection, heredity, immune abnormality, abnormality of neurotransmitter etc., they have not been clarified. In recent years, similarity between fibromyalgia and a disease condition of SART stressed animals has been suggested.

Meanwhile, a mechanism of activation of descending pain control system has been reported as a mechanism in which an extract from inflamed tissues inoculated with vaccinia virus exerts an analgesic action. The inventors of the present invention have investigated neural tissues (dorsal root ganglion, spinal dorsal horn and brain tissues) in a SART stressed rat using a real-time PCR for a purpose of further clarification of a disease condition due to SART stressing and a mechanism of a pharmacological activity of an extract from inflamed tissues inoculated with vaccinia virus. However, a limited number of genes can be analyzed using real-time PCR, therefore, all genes on the amount of which an extract from inflamed tissues inoculated with vaccinia virus can influence have not been explored comprehensively.

Non-Patent Document 1: Kiso To Rinsho (Clinical Report), Vol. 15, No.5, p. 2459, 1981.
Non-Patent Document 2; Ouyou Yakuri (Pharmacametrics), Vol. 32, No. 3, p. 599, 1986.
Patent Document 1: International Publication WO2004/039383

### DISCLOSURE OF THE INVENTION

### [Problem to be Solved by the Invention]

The objective of the present invention is to provide a method for study, determination, or evaluation of a disease condition due to SART stressing and a pharmacological activity, for example an analgesic activity, an autonomic imbalance-ameliorating activity and an anti-stress activity, of a test substance at the gene level as well as a method for screening a substance effective against painful diseases, autonomic imbalance, and stress-related diseases.

### [Means for Solving the Problem]

The inventors of the present invention have carried out intensive studies by paying their attention to the mechanism that an analgesic action of an extract from inflamed tissues inoculated with vaccinia virus on a SART stressed animal improves dysfunction of a descending pain control system due to stress. As a result, they completed the invention by identifying the genes on the amount of which a SART stressing and a test substance can influence through comprehensive exploration of gene expression in the neural tissue of a SART stressed animal that was administered with the test substance .

### [Effects of the Invention]

The present invention provides a method for study, determination or evaluation of a pharmacological activity of a test substance, especially an analgesic activity, an autonomic imbalance-ameliorating activity, or an anti-stress activity at the gene level by comprehensively analyzing the change in gene expression in a neural tissue after administration of a test substance in a SART-stressed animal, enabling exploration of the substance that is effective against painful diseases, autonomic-imbalance, stress-related diseases or the like, determination and evaluation of efficacy of the substance, or analysis of a target gene of the substance.

### BEST MODES FOR CARRYING OUT THE INVENTION

A SART stressed animal can be prepared according to the method described above. An extract from inflamed tissues inoculated with vaccinia virus, or a test substance, is prepared in such a manner that vaccinia virus is inoculated to an animal, the inflamed tissue are ground, an extracting solvent is added thereto, the tissue pieces are removed, a treatment for removal of protein is conducted followed by adsorbing with an adsorbent and the adsorbed component is eluted.

An extract from inflamed tissues inoculated with vaccinia virus is manufactured, for example, by the following steps.
(a) Inflamed skin tissues of rabbits, mice, etc. by inoculation of vaccinia virus are collected, the inflamed tissues are ground, an extracting solvent such as water, aqueous phenol, physiological saline solution or phenol-added aqueous glycerol is added and the mixture is filtered or centrifuged to give an extract fluid (filtrate or supernatant liquid).
(b) The above extracted fluid is adjusted to an acidic pH and heated for deproteinization. Next, the deproteinized solution is made alkaline and heated, and then filtered or centrifuged.
(c) The resulting filtrate or supernatant fluid is made acidic and adsorbed with an adsorbent such as active carbon or kaolin.
(d) An extracting solvent such as water is added to the above adsorbent followed by adjusting to an alkaline pH, and the adsorbed component is eluted whereupon an extract from inflamed tissues inoculated with vaccinia virus is able to be prepared.

Each of the above steps will now be illustrated in more detail as follows.
About (a):
Inflammatory skin tissues where smallpox occurred by inoculation of vaccinia virus to rabbits such as domestic rabbits are collected and aground, and an extracting solvent in 1- to 5-fold amount was added thereto to prepare an emulsified suspension. As to the extracting solvent, distilled water, physiological saline solution, weakly acidic to weakly basic buffer, etc. may be used and a stabilizer such as glycerol, a bactericide/antiseptic agent such as phenol, a salt such as sodium chloride, potassium chloride, and magnesium chloride, etc. may be appropriately added thereto. It is also possible that, at that time, a treatment by means of freeze-thawing, ultrasonic wave, cell membrane dissolving enzyme, surfactant, etc. is conducted to destroy the cellular tissues whereby the extraction is made easy.

About (b):
The resulting milky extract is filtrated, centrifuged or the like to remove the tissue pieces and the a deproteinizing treatment is carried out. The deproteinizing operation is able to be carried out by a known method which has been commonly done and it is possible to apply a method such as a heating treatment, a treatment using a protein modifier such as acid, base, urea, guanidine or an organic solvent such as acetone, an isoelectric precipitation, and a salting out. After that, a common method for removal of insoluble matter such as filtration using filter paper (cellulose, nitrocellulose, etc.), glass filter, Celite, Seitz filter plate, etc., ultrafiltration, or centrifugation is carried out whereby the insoluble protein separated out therefrom is removed.

About (c):
The thus-obtained extract fluid containing the effective component is adjusted to acidic or, preferably, to pH 3.5 to 5.5 using an acid such as hydrochloric acid, sulfuric acid, or hydrobromic acid and an adsorbing operation with an adsorbent is carried out. As to the adsorbent which is able to be used, active carbon, kaolin, etc. may be exemplified and the adsorbent is added to the extract followed by stirring or the extract is passed through a column filled with the adsorbent so that the effective component is able to be adsorbed with the adsorbent. When an adsorbent is added to the extract, the solution is removed by filtration, centrifugation, etc. whereby an adsorbent with which an effective component is adsorbed is able to be prepared.

About (d):
In the elusion (detachment) of the effective component from the adsorbent, it is able to be achieved in such a manner that an eluting solvent is added to the above adsorbent, elation is conducted at room temperature or with appropriate heating or with stirring and the adsorbent is removed by a common method such as filtration or centrifugation. As to the eluting solvent used therefor, a basic solvent such as water, methanol, ethanol, isopropanol or an appropriate mixture thereof which is adjusted to a basic pH, or, preferably, water that is adjusted to a pH 9 to 12, is able to be used.

A manufacturing method of an extract from inflamed tissues inoculated with vaccinia virus is described more specifically in, for example, the above Patent Document 1, Animal neural tissues such as dorsal root ganglion and spinal dorsal horn may be sampled according to an ordinary procedure.

### Examples

### 1. Materials of an experiment

First, neural tissue samples of SART stressed animals were prepared as follows.

### (1) Animals

Male Wister rats of six weeks age were loaded with SART stress to prepare SART stressed rats. The rats were freely fed with feed and tap water and loaded with repeated cold stress for 5 days and, on the sixth day, they were released from the stress load and subjected to the experiment.

### (2) Extract from inflamed tissues inoculated with vaccinia virus

As an extract from inflamed tissues inoculated with vaccinia virus, there was used an extract prepared from the inflammatory skin of rabbit inoculated with vaccinia virus as manufactured according to Example 2 of the above-mentioned Patent Document 1 which was adjusted to 20 NU/mL (an extract from inflamed cutaneous tissues of rabbits inoculated with vaccinia virus). NU is stipulated by ED₅₀ value of analgesic effect when an START stressed mouse which is a chronic stressed animal where pain threshold value is lower than that in normal animal was used and a test according to a modified Randall-Selitto method was conducted. 1 NU is the activity showing 1 mg of an analgesic activity-containing component of the extract from inflamed cutaneous tissues of rabbits inoculated with vaccinia virus when ED₅₀ value is 100 mg/kg.

### (3) Administration of an extract from inflamed cutaneous tissues of rabbits inoculated with vaccinia virus

An extract from inflamed cutaneous tissues of rabbits inoculated with vaccinia virus was intraperitoneally administered once per day consecutively in the dose of 200 NU/kg body weight to the above SART stressed rat from the initial SART stressing day (a normal group administered with test substance and a SART stressed group administered with test substance). A physiological saline solution was administered in the same schedule to a normal control group and to a SART stressed control group. The administered liquid amount was made 10 mL per kg body weight. Groups are organized as follows: the normal control group (n = 3), the normal group administered with test substance (n=3), the SART stressed control group (n = 4), and the SART stressed group administered with the test substance (n = 4).

### (4) Measurement of pain threshold value

The pain threshold value was measured by a test according to a modified Randall-Selitto method using a measuring apparatus for analgesic effect for pressed stimulation. Thus, pressed stimulation was applied to the right hind paw of a rat with a predetermined pressing velocity and the pressing weight (g) by which the animal shows escape reaction or squealing reaction was measured as a pain threshold value. After the end of 5-day SART stressing, final administration of the extract from inflamed cutaneous tissues of rabbits inoculated with vaccinia virus was conducted and the pain threshold value was measured after 30 minutes from the final administration.

From the above, the following result was achieved.

### (1) Effect of an extract from inflamed cutaneous tissues of rabbits inoculated with vaccinia virus on lowering of pain threshold value of SART stressed rats

The pain threshold value of the SART stressed control group whereon a SART stress was loaded for 5 days was significantly reduced compared with that of the normal control group. The result of measurement of the pain threshold value after 30 minutes from the final administration to the SART stressed group administered with the test substance was observed to be significantly improved compared with that of the SART stressed control group. In the meanwhile, no change in the pain threshold was observed in the normal control group whereto an extract from inflamed cutaneous tissues of rabbits inoculated with vaccinia virus was administered without SART stressing.

### (2) Samples

As described above, after the reduction in the pain threshold value due to SART stressing and the analgesic effect of the extract from inflamed cutaneous tissues of rabbits inoculated with vaccinia virus were confirmed, decapitation of the rats in every group was conducted to collect blood and the dorsal root ganglion and spinal dorsal horn were removed out. They were immerses in DNA later (trade name, the product of Ambion), incubated for one night in a refrigerator to prevent decomposition of RNA, and cryopreserved at -80°C.

### 2. Experimental procedure

Next, the amount of gene expression in the dorsal root ganglion (DRG) and spinal dorsal horn (DH) of the SART stressed animals was comprehensively determined with DNA arrays (DNA microarrays, DNA tips, etc.) using the samples obtained in accordance with the above-mentioned method to clarify pathological significance of the change in gene expression. Details are as follows.

### (1) Preparation of total RNA and quality assay

Total DNA of each sample was extracted and purified using RNeasy Lipid Tissue Mini Kit (trade name, the product of Qiagen) and RNase-Free DNase Set (trade name, the product of Qiagen) and then RNA quality assay was done using 2100 Bioanalyzer (trade name, the product of Agilnet), The ratios of absorbance (260/280) of total RNA were 2.0-2.1 among all samples, indicating that every sample was the nucleic acid fraction without protein. Analysis using microelectrophoresis for decomposition of DNA revealed a sharp peak of 18S ribosome RNA in every sample. Also, no irregularity of the baseline or peak of any degradation product showed that they are RNA samples without decomposition. Total RNA in these samples was appropriate for any of quality assays and, therefore, was chosen to be used as a sample for DNA array experiment.

### (2) DNA array experiment (preparation of Cyanine 3 labelled complementary RNA (Cy3-cRNA) and Hybridization)

Rat Genome Oligo Microarray Kit (trade name, the product of Agilent, Product Number G4131 A) was used for a comprehensive analysis of gene expression. Preparation of targets, hybridization, and cleansing where done in accordance with the Agilent's protocol of DNA microarray kit, one-color (Ver 1.0).
After double-strand complementary DNA was prepared through reverse transcription reaction of total RNA, amplification and labeling were done using Low RNA Input Linear Amplification & Labeling Kit in the presence of Cy3-labeled cytosine triphosphate (Cy3-CTP) and Cy3-cRNA was purified using RNeasy Mini kit (trade name, the product of Qiagen).

Concentration and fluorescent intake of the samples were determined using U-3300 Spectrophotometer (trade name, the product of Hitachi), revealing that Cy3-cRNA was amplified to 1000-ford or more and that the rate of Cy3 intake in all samples was ≥9 pmol/µg or more that was fallen within the reference range (9-15 pmol/µg) shown in the Agilent's protocol. Therefore, amplification efficiency and rate or fluorescent intake of Cy3-cRNA prepared by Total RNA were within the reference ranges of the protocol. Thus, amplification efficiency and fluorescent intake were confirmed to be performed appropriately.

Cy3-cRNA obtained as above was fragmented using a reagent attached to Gene Expression Hybridization Kit (trade name, the product of Agilent), mixed with Hybridization Buffer, mounted onto a Rat Genome Oligo Microarray slide, and hybridized at 65°C for 17 hours. The amount of Cy3-cRNA complementarily bound to each probe on the DNA microarray that was cleaned and dried after the reaction was determine as a fluorescence intensity using DNA Microarray Scanner (trade name, the product of Agilent, G2565BA) with a resolution of 10µm.

### (3) Data analysis

### [1] Numerical conversion of the fluorescence intensity and data processing

Numerical conversion of the fluorescence intensity within a certain range in each spot and detection of abnormal spot(s) were done using a numerical conversion software Feature Extraction Ver 8.5 (trade name, the product of Agilent) to eliminate spot(s) with statistically non-significant difference compared with the background value.

### [2] Correction of the amount of gene expression and narrowing of target gene family

After the level of gene expression was corrected among arrays and probes using a gene expression analysis software Gene Spring Ver 7.3 (trade name, the product of Silicon Genetics), exclusion of genes showing abnormal expression pattern and selection of target genes were done as follows,
(A) Selection based on the expression pattern (Flag): genes with abnormal Flag were eliminated.
(B) Selection of genes based on the significant difference in the level of gene expression resulted from analysis of variance: Using analysis of variance (one way ANOVA) among the normal control group (n = 3), the SART stressed control group (n = 4), and the SART stressed group administered with the test substance (n = 4), genes showing the significant (p < 0.05) difference were selected.
(C) Selection of target gene families: Gene families whereof gene expression was increased to 1.25-fold or more in the SART stressed group compared with the normal control group and was decreased to 1.25-fold or more in the SART stressed group administered with the test substance compared with the SART stressed group were selected to narrow target genes by combining START stressing and an effect of the test substance on gene expression. Contrary to this, gene families whereof gene expression was decreased to 1.25-fold or more in the START stressed group compared with the normal control group and was increased to 1.25-fold or more in the SART stressed group administered with the test substance compared with the SART stressed group were selected.

### [3] Estimation of target genes

The function of selected target genes was investigated using a gene database GeriBank and a literature database PubMed.

### 3. Results

The results of the above-mentioned experimental procedure were as follows.

### (1) Selection of target gene families

The amount of fluorescence emitted by genes bound to 41,071 probes on the DNA array was numerically converted and genes with abnormal expression pattern (Flag) were eliminated. Among remaining gene families (DRG-Flag: 19,371 genes, DH-Flag: 20,623 genes), genes showing significant (p < 0.05) difference after the analysis of variance (one way ANOVA) among 3 groups including the normal control group, the SART stressed control group, and the SART stressed group administered with the test substance were selected (DRG-ANOVA; 1,538 genes, DH-ANOVA: 3,570 genes). From the gene families selected after the analysis of variance, the following gene families in which the change in the amount of expression was observed in each sample were selected in accordance with the above procedure for selecting target gene families and listed in Tables 1 to 5. In the following Tables, (-) represents a molecule with no gene symbol and (predicted) represents a predicted molecule. Also, a SART value represents the expression amount in the SART stressed group when the value in the normal control is 1.00 and a SART-NSP value represents the expression amount in the SART stressed group administered with the test substance when the value in the SART stressed group is 1.00.

[1] In the dorsal root ganglion (DRG), 37 genes showing the change (increase in the expression due to SART stressing and decrease in the expression due to the test substance) in the expression amount (Tables 1 and 2)

**Table 1**

| No. | Gene Symbol | Probes | SART | SART-NSP |
|---|---|---|---|---|
| 1 | Cc16 | A_43_P16707 | 1.55 | 0.73 |
| 2 | Cc17 | A_44_P1022002 | 1.51 | 0.68 |
| 3 | Cd74 | A_44_P549494 | 1.65 | 0.78 |
| 4 | RT1-Da | A_44_P991532 | 1.81 | 0.77 |
| 5 | Msr2 (predicted) | A_43_P19999 | 1.53 | 0.56 |
| 6 | (-) | A_44_P340587 | 1.79 | 0.70 |
| 7 | Mst1 | A_43_P12418 | 2.46 | 0.68 |
| 8 | Vcam1 | A_42_P499158 | 1.42 | 0.79 |
| 9 | Cldn1 | A_44_P285463 | 2.35 | 0.64 |
| 10 | Cfh | A_44_P160877 | 1.84 | 0.78 |
| 11 | Tbxas1 | A_44_P260072 | 1.58 | 0.62 |
| 12 | Myh4 (predicted) | A_42_P708941 | 1.38 | 0.40 |
| 13 | Map3k8 | A_43_P15587 | 1.42 | 0.75 |
| 14 | Ptpn7 | A_42_P653257 | 1.64 | 0.78 |
| 15 | Gja1 | A_44_P393551 | 1.39 | 0.79 |
| 16 | Cp | A_44_P404836 | 2.01 | 0.73 |

**Table 2**

| No. | Gene Symbol | Probes | SART | SART-NSP |
|---|---|---|---|---|
| 17 | (-) | A_44_P315727 | 1.44 | 0.80 |
| 18 | Tagln | A_44_P360772 | 1.47 | 0,76 |
| 19 | Ecm2 (predicted) | A_44_P126261 | 1.63 | 0.75 |
| 20 | Mfap5 (predicted) | A_43_P14973 | 2.09 | 0.72 |
| 21 | (-) | A_42_P610733 | 1.38 | 0.55 |
| 22 | Gcgr | A_42_P621872 | 1.39 | 0.76 |
| 23 | (-) | A_44_P530637 | 1.41 | 0.78 |
| 24 | Igfbp5 | A_14_P264240 | 1.48 | 0.79 |
| 25 | (-) | A_44_P915303 | 1.50 | 0.67 |
| 26 | (-) | A_42_P598365 | 1.51 | 0.75 |
| 27 | (-) | A_44_P1060244 | 1.55 | 0.63 |
| 28 | Csh111 | A_44_P391196 | 1.56 | 0.52 |
| 29 | (-) | A_44_P187246 | 1.61 | 0.71 |
| 30 | (-) | A_43_P10874 | 1.64 | 0.69 |
| 31 | Apobec1 | A_42_P598412 | 1.68 | 0.65 |
| 32 | Slc38a6 | A_44_P149267 | 1.75 | 0.79 |
| 33 | Srpx | A_44_P45073 | 1.76 | 0.80 |
| 34 | (-) | A_44_P338743 | 1.84 | 0.76 |
| 35 | (-) | A_44_P1053951 | 1.98 | 0.75 |
| 36 | (-) | A_44_P715240 | 2.09 | 0.76 |
| 37 | (-) | A_44_P635423 | 2.67 | 0.71 |

[2] In the dorsal root ganglion, 12 genes showing the change (decrease in the expression due to SART stressing and recovery of the expression due to the test substance) in the expression amount (Table 3)

**Table 3**

| No. | Gene Symbol | Probes | SART | SART-NSP |
|---|---|---|---|---|
| 1 | Basp 1 | A_43_P12208 | 0.54 | 1.65 |
| 2 | "Odf1 (predicted) | A_44_P429006 | 0.64 | 1.51 |
| 3 | Co12a1 | A_43_P15252 | 0.68 | 1.26 |
| 4 | Hoxd1 (predicted) | A_44_P475829 | 0.60 | 1.42 |
| 5 | RGD1310117 (predicted) | A_44_P550992 | 0.60 | 1.43 |
| 6 | (-) | A_44_P484589 | 0.62 | 1.31 |
| 7 | (-) | A_44_P497985 | 0.67 | 1.59 |
| 8 | (-) | A_44_P113421 | 0.70 | 1.31 |
| 9 | Rasd2 | A_42_P54071 | 0.71 | 1.45 |
| 10 | (-) | A_43_P20615 | 0.72 | 1.32 |
| 11 | (-) | A_44_P305961 | 0.74 | 1.55 |
| 12 | Apln | A_43_P12613 | 0.79 | 1.36 |

[3] In the spinal dorsal horn (DH), 10 genes showing the change (increase in the expression due to SART stressing and decrease in the expression due to the test substance) in the expression amount (Table 4)

**Table 4**

| No. | Gene Symbol | Probes | SART | SART-NSP |
|---|---|---|---|---|
| 1 | (-) | A_44_P161208 | 1.49 | 0.72 |
| 2 | (-) | A_43_P23266 | 1.59 | 0.73 |
| 3 | Slfu2 (predicted) | A_44_P469113 | 1.27 | 0.79 |
| 4 | (-) | A_42_P840729 | 1.29 | 0.76 |
| 5 | | A_44_P538882 | 1.32 | 0.79 |
| 6 | Csh1l1 | A_44_P391196 | 1.44 | 0.56 |
| 7 | Slc13a2 | A_42 _P754715 | 1.81 | 0.73 |
| 8 | Olr1455 (predicted) | A_44_P540755 | 1.87 | 0.67 |
| 9 | (-) | A_44_P197128 | 1.90 | 0.75 |
| 10 | Fbx17 (predicted) | A_44_P137273 | 2.05 | 0.79 |

[4] In the spinal dorsal horn, 27 genes showing the change (decrease in the expression due to SART stressing and recovery of the expression due to the test substance) in the expression amount (Table 5)

**Table 5**

| No. | Gene Symbol | Probes | SART | SART-NSP |
|---|---|---|---|---|
| 1 | (-) | A_43_P16493 | 0.26 | 1.64 |
| 2 | Mpz | A_43_P11783 | 0.33 | 1.52 |
| 3 | Prx | A_42_P353918 | 0.39 | 1.47 |
| 4 | Pmp22 | A_44_P353315 | 0.45 | 1.31 |
| 5 | Myoc | A_42_P547246 | 0.34 | 1.59 |
| 6 | (-) | A_42_P778184 | 0.49 | 1.32 |
| 7 | Vim | A_42_P509365 | 0.61 | 1.26 |
| 8 | Nes | A_44_P1020428 | 0.67 | 1.25 |
| 9 | Glra1 | A_44_P146851 | 0.65 | 1.55 |
| 10 | Kctd5 (predicted) | A_44_P356812 | 0.70 | 1.26 |
| 11 | Prrx2 (predicted) | A_42_P655047 | 0.59 | 1.33 |
| 12 | Hoxd1 (predicted) | A_44_P475829 | 0.78 | 1.27 |
| 13 | Lect1 | A_42_P631818 | 0.23 | 1.29 |
| 14 | RGD1310955(predicied) | A_44_P405302 | 0.44 | 1.40 |
| 15 | Wisp1 | A_42_P816427 | 0.46 | 1.34 |
| 16 | Plekha4 | A_44_P1052067 | 0.53 | 1.37 |
| 17 | Smoc2 (predicted) | A_42_P812805 | 0.57 | 1.50 |
| 18 | Cdc2a | A_42_P580844 | 0.60 | 1.35 |
| 19 | (-) | A_44_P194591 | 0.60 | 1.49 |
| 20 | Pf4 | A_42_P508984 | 0.62 | 1.69 |
| 21 | (-) | A_44_P938669 | 0.63 | 1.35 |
| 22 | (-) | A_44_P344599 | 0.69 | 1.27 |
| 23 | (-) | A_44_P546170 | 0.70 | 1.26 |
| 24 | (-) | A_44_P603700 | 0.73 | 1.30 |
| 25 | (-) | A_44_P588355 | 0.73 | 1.38 |
| 26 | (-) | A_44_P446817 | 0.77 | 1.28 |
| 27 | Ada | A_44_P434541 | 0.78 | 1.43 |

### (2) Functional analysis of target genes

The function of selected target genes as above was investigated using a gene database GenBank and a literature database PubMed, and similar functions were summarized into Tables 6 to 11. In the following Tables, (-) represents a molecule with no gene symbol and (predicted) represents a predicted molecule.

[1] In 37 genes showing in the dorsal root ganglion, the change (increase in the expression due to SART stressing and decrease in the expression due to the test substance) in the expression amount, it was found that genes having functions involved in macrophage chemotaxis and activation and inflammation were included (Tables 6 and 7).

**Table 6**

| No. | Gene Symbol | Functions (number) | Descriptions |
|---|---|---|---|
| 1 | Ccl6 | Chemokine (2) | Chemokine (C-C motif) ligand 6: chemotaxis, apoptosis, proliferation, regression |
| 2 | Ccl7 | | Chemokine (C-C motif) ligand 7: signal, chemotaxis, activation, migration, induction of macrophage daring inflammation and metastasis |
| 3 | Cd74 | antigen presentation (2) | CD74 antigen (invariant polpypeptide of major histocompatibility class II antigen-associated): involved in antigen presentation, involved in differentiation, selection, activation, proliferation etc. Control of MHC class II |
| 4 | RT1-Da | | RA1 class II, locus Da: MHC class II activity, expressing in antigen presenting cells |
| 5 | Msr2 (predicted) | Macrophage activation (3) | Macrophage scavenger receptor 2 (predicted) |
| 6 | (-) | | Macrophage expressed genre 1 |
| 7 | Mst1 | | Macrophage stimulating 1 (hepatocyte growth factor-like): migration, proliferation, morphology, stimulation, activation, scattering |
| 8 | Vcam1 | Cell adhesion (2) | vascular cell adhesion molecule 1: involved in adhesion to cells. Expressing in endothelial cells |
| 9 | Cldn1 | | Claudin 1: involved in formation, permeability, and adhesion. Structure molecule activity, involved in calcium-dependent cell-cell adhesion |
| 10 | Cfh | Inflammation ratings (2) | Complement component factor H: complement activation, cell death |
| 11 | Tbxas1 | | Thromboxane A synthase 1: thromboxane biosynthesis, electron transport, biosynthesis of fatty acid, blood coagulation |
| 12 | Myh4 (predicted) | Signal transduction (3) | Myosin, heavy polypeptide 4, skeletal muscle (predicted): catenin (cadherin-associated protein), involved in PI3K/AKT signal and Writ&beta-catenin signal, apoptosis, proliferation, differentiation, adhesion, colony formation |
| 13 | Map3k8 | | Mitogen-activated protein kinase kinase kinase 8 : metastasis, survival, conversion of G2/M, over-proliferation. Activating MAP kinase and JNK. involved in production of NF-kB in the nucleus |
| 14 | Ptpn7 | | Protein tyrosine phosphatase, non-receptor type 7: involved in cell proliferation differentiation, division, and malignant alteration |
| 15 | Gja1 | Transporter (2) | Gap junction membrane channel protein alpha 1: ion transporter, showing signal transduction activity |
| 16 | Cp | | Ceruloplasmin: transporter of metal-binding protein copper ion where in blood copper is bound. A lack of this molecule causes iron accumulation, leading to damage to tissues. Having ferroxidase activity |

**Table 7**

| No. | Gene Symbol | Functions (number) | Descriptions |
|---|---|---|---|
| 17 | (-) | Cytoskeleton formation (4) | Similar to 60S ribosomal protein L26 |
| 18 | Tagln | | Transgelin: actin binding, muscle growth, unknown function |
| 19 | Ecm2 (predicted) | | Extracellular matrix protein 2, female organ and adipocyte specific (predicted) |
| 20 | Mfap5 (predicted) | | Microfibrillar associate protein 5 (predicted): microfiber-relating glycoprotein, involved in construction of extracellular matrix structure |
| 21 | (-) | Others (17) | Similar to Pellino protein homolog 2 (Pellino 2) |
| 22 | Gcgr | | Glucagon receptor |
| 23 | (-) | | Rattus norvegicus GDP-mannose pyrophosphorylase A (Gmppa), mRNA |
| 24 | Igfbp5 | | Insulin-like growth factor binding protein 5: growth, apoptosis, migration, proliferation, differentiation |
| 25 | (-) | | Q91842 (Q91842) Xenopus laevis U2 snRNA gene, partial (6%) [TC523154] |
| 26 | (-) | | Synonym: binding to carbohydrate, scavenger receptor activity |
| 27 | (-) | | Rattus norvegicus similar to RIKEN cDNA 2310032K21 (LOC362830), mRNA [XM_343158] |
| 28 | Csh111 | | Chorionic somatomammotropin hormone 1-like 1 |
| 29 | (-) | | Similar to cell surface receptor FDFACT |
| 30 | (-) | | Rattus norvegicus similar to tropomyosin 1, embryonic fibroblast-rat (MGC109519), mRNA |
| 31 | ApobecI | | Apolipoprotin B editing complex 1: mRNA editing, mRNA process, metabolism of limpid |
| 32 | Slc38a6 | | Solute carrier family 38, member 6: transporter family |
| 33 | Srpx | | Sushi-repeat-containing protein: apoptosis |
| 34 | (-) | | Gelsolin |
| 35 | (-) | | Rattus norvegicus immediate early response 5-like (Ier51), mRNA |
| 36 | (-) | | MMU 400844 cell surface receptor FDF03 {Mus musculus:}, partial (11 %) [TC520009] |
| 37 | (-) | | Hypothetical gene supported by NM_133555 |

[2] In 12 genes showing in the dorsal root ganglion, the change (decrease in the expression due to SART stressing and recovery of the expression due to the test substance) in the expression amounts it was found that genes having functions involved in cell growth were included (Table 8).

**Table 8**

| No. | Gene Symbol | Functions (number) | Descriptions |
|---|---|---|---|
| 1 | Basp1 | Cell growth (3) | Brain abundant, membrane attached signal protein1: over-growth, germination, Cytoskeleton formation |
| 2 | Gdf1 (predicted) | | Growth differentiation factor 1 (predicted): controlling cell proliferation and differentiation. Involved in growth of nerves |
| 3 | col2a1 | | Procollagen, type II, alpha 1: aggregation, migration, binding, adhesion, degranulation, growth, and proliferation. Structuring extra cellular matrix |
| 4 | Hoxd1 (predicted) | Others (9) | Homeo box D1 (predicted): control of transcription, localized in the nucleus, involved in differentiation and growth of extremities (limbs) |
| 5 | RGD1310117 (predicted) | | Hypothetical LOC298591 (predicted) |
| 6 | (-) | | Cystatin TE-1 |
| 7 | (-) | | Similar to oxysterol-binding protein-like protein 10 |
| 8 | (-) | | Rattus norvegicus N-myristoyltransferase 2 (Nmt2), mRNA |
| 9 | Rasd2 | | RASD family, member 2 |
| 10 | (-) | | Similar to high density lipoprotein-binding protein |
| 11 | (-) | | Related RAS viral (r-ras) oncogene homolog 2 (predicted) |
| 12 | Apln | | Apelin, AGTRL1 ligand: hormone activity, showing chemotaxis. Involved in immune response, signal transduction, and lactation. Involved in hypertension and diueresis |

[3] In 10 genes showing in the spinal dorsal horn, the change (increase in the expression due to SART stressing and decrease in the expression due to the test substance) in the expression amount, it was found that genes having functions involved in cell adhesion were included (Table 9).

**Table 9**

| No. | Gene Symbol | Functions (number) | Descriptions |
|---|---|---|---|
| 1 | (-) | Cell culture (2) | Similar to R-cadherin |
| 2 | (-) | | Rattus norvegicus protocadherin beta 21 (predicted) (Pcdhb21_predicted), mRNA [NM_001014788] |
| 3 | Slfn2 (predicted) | Others (8) | Schlafen 2 (predicted): unknown function |
| 4 | (-) | | Transcribed locus |
| 5 | (-) | | Similar to Dynamic binding protein (Scaffold protein Tuba) |
| 6 | Cshlll | | Chorionic somatomammotropin hormone 1-like 1 |
| 7 | Slc13a2 | | Solute carrier family 13 (sodium-dependent dicarboxylate transporter), member 2 |
| 8 | Olr1455 (predicted) | | Olfactory receptor 1455 (predicted): G protein binding receptor, olfactory receptor, interacting with odor molecule to initiate nerve reaction for recognizing the odor |
| 9 | (-) | | Rattus norvegicus similar to Zinc finger protein 7 (Zinc finger protein KOX4) (Zinc finger protein HF.16) (LOC362947), mRNA [XM_343277] |
| 10 | Fbx17 (predicted) | | F-box and leucine-rich repeat protein 7 (predicted): belonging to F-box protein family. Having ubiquitin-dependent ligase activity |

[4] In 27 genes showing in the spinal dorsal horn, the change (decrease in the expression due to SART stressing and recovery of the expression due to the test substance) in the expression amount, it was found that genes having functions involved in myelination, formation of neuroskeleton, and hyperpolarization were included (Tables 10 and 11).

**Table 10**

| No. | Gene Symbol | Functions (number) | Descriptions |
|---|---|---|---|
| 1 | (-) | Myelination (4) | Rattus norvegicus similar to myelin P2 protein |
| 2 | Mpz | | Myelin protein zero: primary constituent of peripheral myelin, involved in synaptic transmission and development of peripheral nerve |
| 3 | Prx | | Periaxin: protein in Schwann cell, having relation to allodynia and hyperalgesia |
| 4 | Pmp22 | | Peripheral myelin protein 22: primary constituent of peripheral myelin, involved in morphology, myelination |
| 5 | Myoc | Cytoskeleton formation (4) | Myocilin: believed to have a role in cytoskeleton function. Trabecular meshwork inducible glucocorticoid response protein |
| 6 | (-) | | Skeletal troponin C: bound with calcium ion. Structural constituent of cytoskeleton, involved in growth of muscle |
| 7 | Vim | | Vimentin: intermediate filament like actin and tubulin. Having structural molecular function |
| 8 | Nes | | Nesitn: intermediate filament protein, expressed in stem cells in the central nerves. Important for growth of the central nerves |
| 9 | Clra1 | Channel (2) | Glycine receptor, alpha 1 subunit: glycine-gated chloride channel, having neurotransmitter receptor activity |
| 10 | Kctd5 (predicted) | | Potassium channel tetramerisation domain containing 5 (predicted): potential-dependent potassium channel |
| 11 | Prrx2 (predicted) | Transcription factor (2) | Paired related homeobox 2 (predicted): having homeodomain. Involved in transcriptional regulation |
| 12 | Hoxd1 (predicted) | | Homo box D1 (predicted) |

**Table 11**

| No. | Gene Symbol | Functions (number) | Descriptions |
|---|---|---|---|
| 13 | Lect1 | Others (15) | Leukocyte cell derived chemotaxin 1: involved in development. Metabolism of proteoglycan, differentiation of cells, constituent of basal membrane |
| 14 | ROD1310955(predicted) | | Similar to carboxypeptidase X 2 (M14 family): carboxypeptidase X2: metallocarboxypeptidase 2 (predicted) |
| 15 | Wisp1 | | WNT1 inducible signaling pathway protein 1 : Binding to insulin-like growth factors. Control or cell proliferation, cell adhesion, signal transduction |
| 16 | Plekha4 | | Pleckstrin homology domain containing, family A (phosphoinositide blinding specific) member 4: having domain binding to phosphatidyl inositol with unknown, function |
| 17 | Smoc2 (predicted) | | SPAR related modular calcium binding 2 (predicted) |
| 18 | Cdc2a | | Cell division cycle 2 homolog A (S. pombe): having cyclin-dependent kinase activity, involved in apoptosis and cell division |
| 19 | (-) | | Similar to hypothetical protein D630003M21 |
| 20 | Pf4 | | Platelet factor 4: involved in chemotaxis, proliferation, activation, differentiation, adhesion. Getting neutrophil and fibroblast together |
| 21 | (-) | | Q6MG84 (Q6MG84) NG3 protein, partial (32%) [TC552370] |
| 22 | (-) | | Similar to transcription enhancer factor |
| 23 | (-) | | Rattus norvegicus similar to Ubiquitin-conjugating enzyme E2H (Ubiquitin-protein ligase H) (Ubiquitin carrier protein H) (UBCH2) (E2-20K) (LOC296956), mRNA [XM_216109] |
| 24 | (-) | | Lysophospholipase |
| 25 | (-) | | Transcribed locus |
| 26 | (-) | | LOC499655 |
| 27 | Ada | | Adenosine deaminase: hydrolyzing adenosine into inosine. Defect of this molecule causes severe immunodeficiency |

As described above, in this experiment, it was found that genes relating to chemokine, Macrophage, cell adhesion, inflammation-related function are highly expressed in the dorsal root ganglion of SART stressed animals. The increase in expression of these genes may be involved in chemotaxis of macrophage cells into the dorsal root ganglion, hyperalgesia due to promotion of their activation, and stress-related diseases. In the meanwhile, it was found that genes realting to myelination and Cl channel function were limitedly expressed in the spinal dorsal horn of SART stressed animals. The decrease in expression of genes involved in myelination may possibly induce demyelinating degeneration that is considered as a cause of hyperalgesia, while the decrease in expression of genes involved in Cl channel function that induces hyperpolarization is strongly related to occurrence of hyperalgesia. This experiment suggested that the extract from inflamed cutaneous tissues of rabbits inoculated with vaccinia virus may exert analgesic action and anti-stress action through controlling expression of these gens.

Therefore, under the conditions of the experiment above, genes which expression was found to vary after SART stressing and administration of the extract from inflamed cutaneous tissues of rabbits inoculated with vaccinia virus may possibly be genes involved in physiological dysfunction such as decrease in the pain threshold in a SART stressed animal or pathogenesis of stress-related diseases and possibly be target genes in which the extract from inflamed cutaneous tissues of rabbits inoculated with vaccinia virus is involved. Therefore, when the change of expression of these genes is normalized by the test substance, the test substance may possibly be a drug effective against painful diseases such as low back pain, neck-shoulder-arm syndrome, symptomatic neuralgia, periarthritis scapulohumeralis, degenerative arthritis deformans, post-herpetic neuralgia, RSD, and fibromyalgia, neurogenic pain, autonomic imbalance, or physiological dysfunction due to stress.

### INDUSTRIAL APPLICABILITY

As described above, the present invention was confirmed to enable detection and identification of genes which expression is changed after SART stressing and administration of an extract from inflamed cutaneous tissues of rabbits inoculated with vaccinia virus. Therefore, the present invention is useful as a method for study, determination or evaluation of a pharmacological activity such as an analgesic activity, an autonomic imbalance-ameliorating activity or an anti-stress activity, of a test substance by analyzing gene expression in a neural tissue after administration of the test substance in a SART stressed animal.

## Claims

1. A method for study, determination, or evaluation of a pharmacologies activity or a test substance by analyzing a gene expression in a neural tissue of a SART stressed animal administered with the test substance.

2. The method for study, determination, or evaluation according to claim 1, wherein the pharmacological activity is an analgesic activity, an autonomic imbalance-ameliorating activity, or an anti-stress activity.

3. The method for study, determination, or evaluation according to claim I or 2, wherein the SART stressed animal is a rat.

4. The method for study, determination, or evaluation according to any one of claims 1 to 3, wherein the neural tissue is a dorsal root ganglion or a spinal dorsal horn.

5. The method for study, determination, or evaluation according to any one of claims 1 to 4, wherein the gene expression analysis is an analysis using DNA arrays.

6. The method for study, determination, or evaluation according to any one of claims 1 to 5, wherein a gene of a SART stressed animal, expression of which is revealed through a gene expression analysis to be changed to a larger degree than a normal animal, is a target gene.

7. The method for study, determination, or evaluation according to any one of claims 1 to 5, wherein a gene of a SART stressed animal which is administered with the test substance, expression of which is revealed through a gene expression analysis to be changed to a larger degree than a SART stressed animal without administration of the test substance, is a target gene.

8. The method for study, determination, or evaluation according to claim 6 or 7, wherein one or two or more of the genes which are listed in Tables 1 to 5 and expression of which are changed are target genes.

9. The method for study, determination, or evaluation according to any one of claims 1 to 8, wherein the test substance is an extract from inflamed tissues inoculated with vaccinia virus.

10. The method for study, determination, or evaluation according to claim 9, wherein the test substance is an extract from inflamed cutaneous tissues of rabbits inoculated with vaccinia virus.

11. A drug studied, determined, or evaluated using the method as claimed in any one of claims 1 to 8.

12. The drug according to claim 11, which is an analgesic drug.

13. The drug according to claim 11, which is an autonomic imbalance-ameliorating drug.

14. The drug according to claim 11, which is an anti-stress drug.

15. The drug according to claim 11, wherein an effective ingredient is an extract from inflamed cutaneous tissues of rabbits inoculated with vaccinia virus.

16. A method for using a SART stressed animal used for the method for study, determination, and evaluation as claimed in claims 1 to 8.

17. The method for using a SART stressed animal used for the method for study, determination, and evaluation as claimed in claims 1 to 8, wherein the test substance is an extract from inflamed cutaneous tissues of rabbits inoculated with vaccinia virus.
